Europäisches Patentamt

European Patent Office

Office européen des brevets

(11) Veröffentlichungsnummer : **0 106 015**
**B1**

(12) **EUROPÄISCHE PATENTSCHRIFT**

(45) Veröffentlichungstag der Patentschrift :
03.12.86

(51) Int. Cl.⁴ : **A 61 M 16/01**

(21) Anmeldenummer : **83105800.3**

(22) Anmeldetag : **14.06.83**

(54) Vorrichtung zur Beimischung flüssiger Narkosemittel in das dem Patienten zuzuführende Atemgas.

(30) Priorität : **17.09.82 DE 3234474**

(43) Veröffentlichungstag der Anmeldung :
**25.04.84 Patentblatt 84/17**

(45) Bekanntmachung des Hinweises auf die Patenterteilung : **03.12.86 Patentblatt 86/49**

(84) Benannte Vertragsstaaten :
**CH FR GB IT LI NL SE**

(56) Entgegenhaltungen :
**DE-A- 2 158 701**
**DE-A- 3 116 951**
**DE-B- 1 271 902**
**DE-B- 1 922 404**

(73) Patentinhaber : **Drägerwerk Aktiengesellschaft**
**Moislinger Allee 53-55**
**D-2400 Lübeck 1 (DE)**

(72) Erfinder : **Albarda, Scato**
**Am Kannenbruch 11**
**D-2061 Gross Schenkenberg (DE)**

**Beschreibung**

Die Erfindung betrifft eine Vorrichtung zur Beimischung flüssiger Narkosemittel in das dem Patienten zuzuführende Atemgas mit einer Steuer- und Auswerteschaltung und Einrichtungen zur geregelten Zuführung des Atemgases sowie des Narkosemittels, mit einer Mischkammer in einem wärmeisolierten Gehäuse mit einem Einlaß für das Atemgas vor, sowie mit einem Auslaß für das Atemgas plus dem verdunsteten Narkosemittel hinter der Mischkammer, wobei der Einlaß einen Einlaßtemperaturfühler und der Auslaß einen Auslaßtemperaturfühler besitzen.

In Geräten der Medizintechnik, bei denen irgendein Versagen zu lebensbedrohenden Umständen für den Patienten führen kann, ist es selbstverständlich, daß Bauteile oder deren Anordnungen von anderen unabhängig funktionierenden Bauteilen überwacht werden oder Verknüpfungen vorhanden sind, bei denen Differenzen zwischen Ist- und Sollwert Signale geben. Mit dem Signal werden visuelle oder auditive Signale ausgelöst und/oder automatische Abhilfemaßnahmen, wie z. B. das Umschalten auf Reservebaugruppen, eingeleitet.

Eine in der älteren Anmeldung DE-AI-3116951 dargestellte Vorrichtung zur Beimischung flüssiger Narkosemittel in das dem Patienten zuzuführende Atemgas dient der Überwachung der Konzentration, des Atemgasdurchflusses und der flüssigen Narkosemittelmenge. Sie nutzt dazu die Erscheinung, daß bei der Verdunstung des dem Atemgas beigemischten flüssigen Narkosemittels eine Temperaturerniedrigung eintritt, die von dem Verhältnis zwischen der verdunsteten Narkosemittelmenge und der Atemgasmenge, also der Konzentration, abhängt. Über einen Einlaß wird das Atemgas zusammen mit dem über eine Zuführungsleitung zugeleiteten Narkosemittel von außen in eine wärmeisolierte Mischkammer eingeführt ; sie mündet tangential in diese, um eine zyklonartige Strömung zu bilden. Vor der Mischkammer bildet die in dem Einlaß geführte Zuführungsleitung einen Wärmeaustauscher. Zwischen dem Wärmeaustauscher und der Mischkammer ragt ein Einlaßtemperaturfühler in den Einlaß. Ein Auslaß für das Gemisch aus dem Atemgas plus dem verdunsteten Narkosemittel führt aus der Mischkammer nach außen zum Patienten. Er enthält hinter der Mischkammer einen Auslaßtemperaturfühler. Die Wand der Mischkammer besitzt eine elektrische Heizung. Die beiden Meßfühler und die Heizung sind mit einer Steuer- und Auswerteschaltung verbunden, die periodisch zwischen zwei Betriebsphasen umschaltet. In der ersten Phase wird die Heizung derart geregelt betrieben, daß Temperaturgleichheit zwischen den beiden Temperaturfühlern besteht. Die zugeführte gemessene Heizleistung entspricht damit der Verdunstungswärme der zugeführten flüssigen Narkosemittelmenge und ist ein Maß für den Narkosemittelfluß. In der zweiten Phase ist die Heizung ausgeschaltet, und es wird bei voller Verdunstung die sich zwischen den beiden Temperaturfühlern einstellende Temperaturdifferenz festgestellt. Sie hängt von dem Verhältnis zwischen verdunsteter Narkosemittelmenge und Atemgasmenge ab, ist also ein Maß für die Konzentration des Narkosemittels im Atemgas. Der Quotient aus den Meßergebnissen beider Phasen bildet ein Maß für den Atemgasdurchfluß, es gilt dann

$$\frac{\text{Narkosemittelfluß}}{\text{Narkosemittelkonzentration}} = \text{Atemgasdurchfluß}$$

(DE-AI-3116951)

Aufgabe der Erfindung ist eine Vorrichtung zur Beimischung flüssiger Narkosemittel in das dem Patienten zuzuführende Atemgas, die mit einfachem Aufbau kurzfristig in konstanter Arbeitsweise einen kontinuierlichen Angleich auch bei Extremforderungen in bezug auf Atemgasmengen und gewünschte Konzentration sichert und dabei die Forderung nach einer sicheren Überwachung durch das Personal und/oder einer automatischen Einleitung von Abhilfen erfüllt.

Die Lösung der Aufgabe erfolgt gemäß dem Kennzeichen des Anspruchs 1. Das Kennzeichen des Anspruchs 2 bestimmt den Aufbau der Steuer- und Auswerteschaltung.

Die Vorrichtung nach der Erfindung bietet die Möglichkeit der Kontrolle des Narkosemittelstromes, des Atemgasstromes und seines Mischverhältnisses sowie der Einhaltung der gewünschten Konzentration. Die Anordnung eines dritten Temperaturfühlers, nämlich des Zwischentemperaturfühlers, zwischen dem Einlaßtemperaturfühler und dem Auslaßtemperaturfühler noch im Einlaß hinter der hier angebrachten Heizung erlaubt, daß die Funktionen mit der Kontrolle des Narkosemittelstromes, des Atemgasstromes, des Mischverhältnisses und der Feststellung der Konzentration kontinuierlich ablaufen können. Die Heizung vor der Mischkammer im Einlaß garantiert die vollständige Verdunstung des flüssigen Narkosemittels.

Dabei sind die Messung der Leistungsaufnahme und der Temperatur der Heizung durch die Feststellung der Spannung und des Stromes sowie die bekannten Temperaturen der drei Temperaturfühler zusammen mit den bekannten Daten des Narkosemittels und der Komponenten des Atemgases die Basis für die Feststellung der kontrollierten Werte.

Weil die Messungen zeitlich gleichzeitig erfolgen, sind alle Meßwerte unter Berücksichtigung der thermischen Zeitkonstante der Vorrichtung jederzeit verfügbar. Es besteht mit den bekannten Änderungsgeschwindigkeiten die Möglichkeit einer Vorhersage.

Eine Ausführung der Erfindung ist im folgenden beschrieben. Dabei zeigen

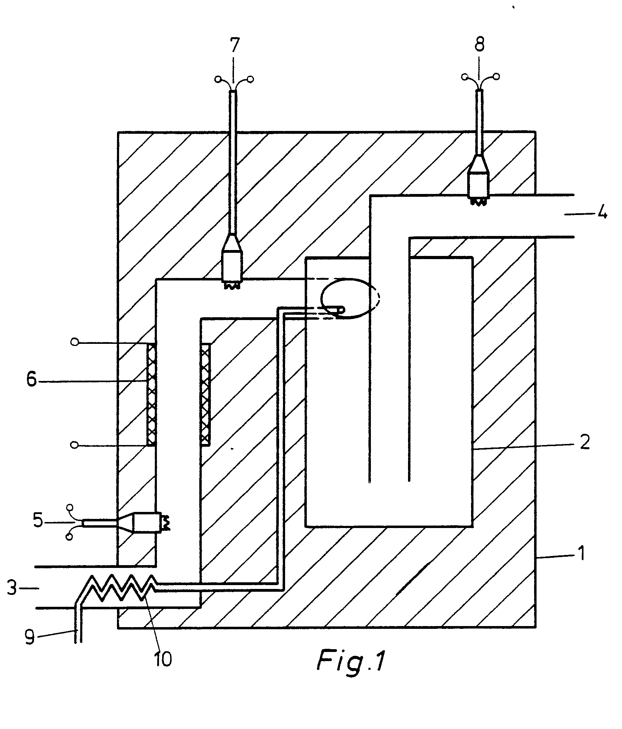

Figur 1   die Mischvorrichtung,
Figur 2   das Schaltschema der Steuer- und Auswerteschaltung.

Ein wärmeisoliertes Gehäuse 1 enthält im Innern eine Mischkammer 2, an die ein Einlaß 3 und ein Auslaß 4 angeschlossen sind. Im Einlaß 3 sind ein Einlaßtemperaturfühler 5, eine Heizung 6 als elektrisches Heizelement sowie dahinter ein Zwischentemperaturfühler 7 und im Auslaß 4 — also hinter der Mischkammer 2 — ein Auslaßtemperaturfühler 8 angeordnet. Eine Zuführungsleitung 9 für ein flüssiges Narkosemittel bildet im Einlaß 3 vor dem Einlaßtemperaturfühler 5 einen Wärmeaustauscher 10 in Form einer Rohrschlange. Die Zuführungsleitung 9 verläuft dann von dort getrennt von dem Einlaß 3 durch das Gehäuse 1. Sie tritt hinter dem Zwischentemperaturfühler 7 wieder in den Einlaß 3 ein und endet mit diesem zusammen in tangentialer Einführung in der Mischkammer 2.

Das Atemgas wird am Einlaß 3 zugeführt und tritt von dort aus dann tangential in die Mischkammer 2 ein. In der sich ausbildenden zyklonartigen Strömung verdunstet das durch die Zuführungsleitung 9 zugeführte flüssige Narkosemittel und mischt sich dabei gleichmäßig mit dem Atemgas. Durch den Wärmeaustauscher 10 angeglichen besitzen das Atemgas und das flüssige Narkosemittel hinter dem Wärmeaustauscher 10 die gleiche Temperatur. Die Temperatur wird durch den Einlaßtemperaturfühler 5 festgestellt. Durch die Heizung 6 wird die Temperatur des Atemgases vor dem Eintritt in die Mischkammer 2 auf eine später noch darzustellende Zwischentemperatur, die durch den Zwischentemperaturfühler 7 gemessen wird, erhöht. Der Auslaßtemperaturfühler 8 mißt die Temperatur des aus der Mischkammer 2 über den Auslaß 4 austretenden Narkosemittel-Atemgas-Gemisches.

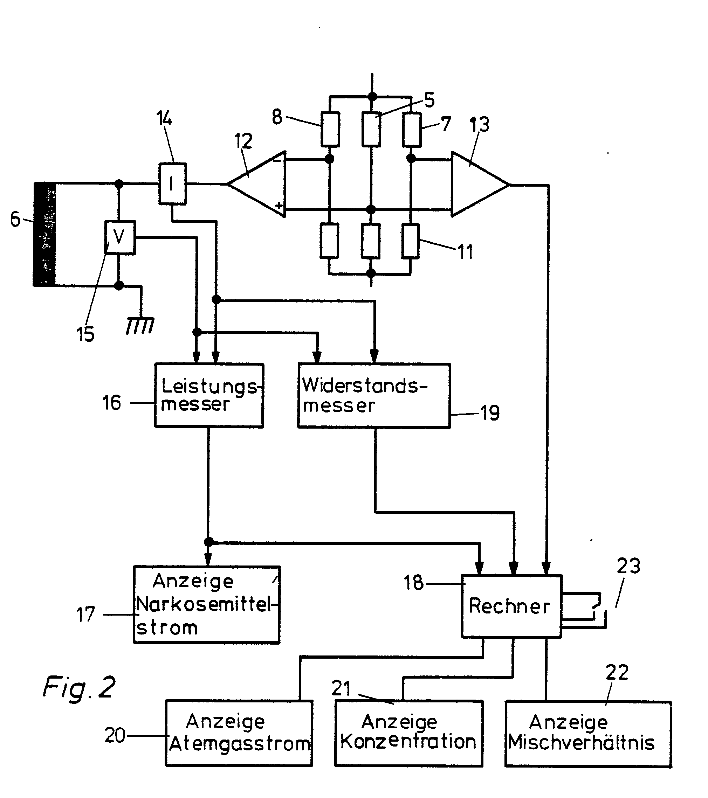

Das in Fig. 2 dargestellte Schaltbild der Steuer- und Auswerteschaltung steuert die oben beschriebene Vorrichtung gemäß folgenden Gedankengängen, wobei
a) der Atemgasstrom $\dot{M}_g$ und
b) der Narkosemittelstrom $\dot{M}_N$
vorgegeben sind,
c) die Temperaturen $T_5$ an dem Einlaßtemperaturfühler 5 und $T_8$ an dem Auslaßtemperaturfühler 8 gleich sind, also $T_5 = T_8$,
d) die Temperatur $T_7$ am Zwischentemperaturfühler 7 gemessen wird,
e) $c_g$ die spezifische Wärme des bekannten Atemgases,
f) $q_f$ die Verdunstungswärme des flüssigen Narkosemittels und
g) $Q_6$ die Leistungsaufnahme der Heizung sind.

Zur Überwachung des Narkosemittelstromes $\dot{M}_N$ wird die Heizung 6 geregelt betrieben, bis sich zwischen dem Einlaßtemperaturfühler 5 und dem Auslaßtemperaturfühler 8 die gleiche Temperatur $T_5$ und $T_8$, also eine Temperaturdifferenz Null einstellt. In diesem Zustand deckt die zugeführte Heizleistung gerade die Verdunstungswärme des zugeführten Narkosemittelstromes $\dot{M}_N$. Eine Beeinflussung von außen ist durch die Wärmeisolierung des Gehäuses 1 ausgeschaltet. Die Messung ist unabhängig vom Atemgasstrom $M_g$, da dessen Menge, Zusammensetzung und Temperatur im Einlaß 3 und Auslaß 4 gleich sind.

Die in Funktion befindliche Heizung 6 heizt den durch den Einlaß 3 fließenden Atemgasstrom $\dot{M}_g$ auf. Die sich mit $T_5 = T_8$ am Zwischentemperaturfühler 7 einstellende Temperatur $T_7$ wird gemessen. Für die Vorrichtung gilt jetzt

$$\dot{M}_N \cdot q_f = Q_6 = \dot{M}_g \cdot c_g \cdot (T_7 - T_5)$$

Die Temperaturfühler 5, 7, 8 sind Widerstandsfühler. Sie sind mit zugehörigen Abgleichwiderständen 11 mit den Eingängen zweier Verstärker 12, 13 verbunden.

Der Verstärker 12 speist die Heizung 6 derart, daß die Temperaturen des Einlaßtemperaturfühlers 5 und des Auslaßtemperaturfühlers 8 gleich werden. In den Leitungen angeordnete Strommesser 14 und Spannungsmesser 15 ergeben über einen Leistungsmesser 16 eine Anzeige 17 des Narkosemittelstromes $\dot{M}_N$.

Das Signal des Leistungsmessers 16 wird zugleich einem Rechner 18 eingegeben. Der Verstärker 13 bildet ein Signal aus der in der Heizung 6 eintretenden Erwärmung des Atemgases, festgestellt zwischen dem Einlaßtemperaturfühler 5 und dem Zwischentemperaturfühler 7, und gibt dieses an den Rechner 18. Im Rechner 18 werden aus diesen Signalen die Werte für den Atemgasstrom $\dot{M}_g$ und mit dem Narkosemittelstrom $\dot{M}_N$ die Konzentration ermittelt und auf Anzeigen 20 und 21 angezeigt.

Werden als Atemgas verschiedene Gasgemische benutzt, dann muß dieses Gemisch und damit seine spezifische Wärme $c_g$ bekannt sein. Die Bestimmung des Mischverhältnisses erfolgt bei bekannten Komponenten (für Narkosezwecke sind die Komponenten Sauerstoff und Lachgas oder Sauerstoff und Luft üblich) aufgrund der Wärmeleitung. Von dieser ist der Wärmeübergang von der Heizung 6 in das Atemgas abhängig, gehört also auch eine andere Temperatur der Heizung zu einer am Zwischentemperaturfühler 7 festgestellten Erwärmung des Atemgases. Die Daten sind dem Rechner 18 in Speichern beigegeben. Ein Umschalter 23 dient der Speicherumschaltung bei einem Wechsel der benutzten Komponenten. Aus den Signalen des Strommessers 14 und des Spannungsmessers 15 bildet ein Widerstandsmesser 19 ein Signal, das dem temperaturabhängigen Widerstand der Heizung 6 entspricht und im Rechner 18 als Maß für die Temperatur der Heizung 6 dient. Aus den gemessenen Werten und den Speicherdaten bestimmt der Rechner 18 dann das vorhandene Mischverhältnis, ausgegeben mit der Anzeige 22 und den Atemgasstrom, ausgegeben mit der Anzeige 20.

**Patentansprüche**

1. Vorrichtung zur Beimischung flüssiger Narkosemittel in das dem Patienten zuzuführende Atemgas mit einer Steuer- und Auswerteschaltung (5-8, 11-13) und Einrichtungen zur geregelten Zuführung des Atemgases sowie des Narkosemittels, mit einer Mischkammer (2) in einem wärmeisolierten Gehäuse (1) mit einem Einlaß (3) für das Atemgas vor, sowie mit einem Auslaß (4) für das Atemgas plus dem verdunsteten Narkosemittel hinter der Mischkammer (2), wobei der Einlaß (3) einen Einlaßtemperaturfühler (5) und der Auslaß (4) einen Auslaßtemperaturfühler (8) besitzen, dadurch gekennzeichnet, daß

a) in dem Einlaß (3) hinter dem Einlaßtemperaturfühler (5) vor dem Eintritt in die Mischkammer (2) eine Heizung (6) und hinter dieser ein Zwischentemperaturfühler (7) angeordnet sind,

b) eine Zuführungsleitung (9) für das Narkosemittel im Einlaß (3) vor dem Einlaßtemperaturfühler (5) einen Wärmeaustauscher (10) bildet und dort dann durch das Gehäuse (1) an der Heizung (6) vorbeiläuft und hinter dem Zwischentemperaturfühler (7) wieder in den Einlaß (3) eintritt und mit diesem zusammen in der Mischkammer (2) endet.

2. Vorrichtung zur Beimischung flüssiger Narkosemittel in das dem Patienten zuzuführende Atemgas nach Anspruch 1, dadurch gekennzeichnet, daß

c) die Leistungsaufnahme der Heizung (6) über einen Strommesser (14) sowie einen Spannungsmesser (15) gemessen, in einem Leistungsmesser (16) festgestellt und dann auf einer Anzeige (17) der Narkosemittelstrom angezeigt wird, während gleichzeitig das Signal einem Rechner (18) anliegt und

d) gleichzeitig die Meßwerte des Strommessers (14) und des Spannungsmessers (15) über einen Widerstandsmesser (19) als dessen Signal und

e) über einen Verstärker (13) die sich aus der Erwärmung des Atemgases im Einlaß (3) durch die Heizung (6) ergebenden Werte der Temperaturdifferenz zwischen dem Einlaßtemperaturfühler (5) und dem Zwischentemperaturfühler (7) dem Rechner (18) anliegen und

f) dem Rechner (18) eine Anzeige (20) für den Atemgasstrom und/oder eine Anzeige (21) für die Konzentration und/oder eine Anzeige (22) für das Mischverhältnis des Atemgases angeschlossen sind.

**Claims**

1. An apparatus, for mixing an anaesthetic liquid with respiratory gas to be supplied to a patient, having : a control and evaluation circuit arrangement (5-8, 11-13) and devices for the regulated supply of the respiratory gas and the anaesthetic ; having a mixing chamber (2), in a thermally insulated housing (1), with an inlet (3) for the respiratory gas before, as well as an outlet (4) for the respiratory gas and the vaporised anaesthetic after, the mixing chamber (2) ; the inlet (3) having an inlet temperature sensor (5) and the outlet (4) having an outlet temperature sensor (8), characterised in that :

a) arranged in the inlet (3), after the inlet temperature sensor (5) and before the entrance into the mixing chamber (2), there is a heating means (6) and after this an intermediate temperature sensor (7) ; and

b) a supply line (9) for the anaesthetic forms a heat exchanger (10) in the inlet (3) before the inlet temperature sensor (5), and then passes through the housing (1) past the heating means (6), and re-enters the inlet (3) after the intermediate temperature sensor (7) and ends together with said inlet in the mixing chamber (2).

2. An apparatus, for mixing an anaesthetic liquid with respiratory gas to be supplied to a patient, as claimed in claim 1, characterised in that :

c) the power consumption of the heating means (6) is measured using a current meter (14) and a voltmeter (15), is calculated in a power meter (16), and is then indicated on a display (17) of the anaesthetic flow, whilst at the same time the signal is applied to a computer (18) ; and

d) at the same time, the measured values from the current meter (14) and the voltmeter (15), as the output signal of an ohmmeter (19) and,

e) *via* an amplifier (13), the values of the temperature difference between the inlet temperature sensor (5) and the intermediate temperature sensor (7), resulting from the rise in temperature of the respiratory gas in the inlet (3) caused by the heating means (6), are applied to the computer (18) ; and

f) a display (20), for the respiratory gas flow, and/or a display (21), for the concentration, and/or a display (22), for the mixture ratio of the respiratory gas, are connected to the computer (18).

**Revendications**

1. Dispositif pour l'incorporation d'un produit anesthésique liquide au gaz respiratoire destiné à un patient, avec un montage de commande et d'évaluation (5-8, 11-13) et des dispositifs pour l'alimentation régulée en gaz respiratoire ainsi qu'en produit anesthésique, avec une chambre de mélange (2) dans un

4

boîtier (1) isolé thermiquement et présentant, en amont de la chambre de mélange (2), une prise d'entrée (3) pour le gaz respiratoire, ainsi que, en aval de la chambre de mélange (2), une prise de sortie (4) pour le gaz respiratoire additionné du produit anesthésique vaporisé, la prise d'entrée (3) possédant une sonde de température d'entrée (5) et la prise de sortie (4) une sonde de température de sortie (8), caractérisé en ce que :

a) un élément chauffant (6), suivi d'une sonde de température intermédiaire (7), sont disposés dans la prise d'entrée (3), après la sonde de température d'entrée (5) et avant l'entrée dans la chambre de mélange (2),

b) une conduite d'alimentation (9) en produit anesthésique forme un échangeur de chaleur (10) dans la prise d'entrée (3), en amont de la sonde de température d'entrée (5), puis passe devant l'élément chauffant (6) à travers le boîtier (1), pour rentrer ensuite dans la prise d'entrée (3) en aval de la sonde de température intermédiaire (7), et déboucher conjointement avec la prise d'entrée (3) dans la chambre de mélange (2).

2. Dispositif pour l'incorporation d'un produit anesthésique liquide au gaz respiratoire destiné à un patient selon la revendication 1, caractérisé en ce que :

c) la puissance absorbée par l'élément chauffant (6) est mesurée par l'intermédiaire d'un ampèremètre (14) ainsi que d'un voltmètre (15), déterminée dans un wattmètre (16) puis affichée sur une unité d'affichage (17) du flux de produit anesthésique, tandis que le signal correspondant est simultanément transmis à un calculateur (18), et que,

d) dans le même temps, les valeurs de mesure fournies par l'ampèremètre (14) et le voltmètre (15) sont, par l'intermédiaire d'un ohmmètre (19), transmises au calculateur (18) sous la forme du signal de sortie de l'ohmmètre (19), et que,

e) toujours dans le même temps, les valeurs de différence de température entre la sonde de température d'entrée (5) et la sonde de température intermédiaire (7), résultant du réchauffement du gaz respiratoire par l'élément chauffant (6) dans la prise d'entrée (3), sont, par l'intermédiaire d'un amplificateur (13), transmises au calculateur (18) ; et en ce que

f) une unité d'affichage (20) du flux de gaz respiratoire et/ou une unité d'affichage (21) de la concentration et/ou une unité d'affichage (22) du rapport de mélange du gaz respiratoire sont reliées au calculateur (18).

Fig.1

Fig. 2